# EUROPEAN PATENT APPLICATION

(11) **EP 4 516 102 A1**
(43) Date of publication of application: **05.03.2025**
(21) Application number: 23796310.3
(22) Date of filing: 24.04.2023
(51) Int. Cl.: A01N 47/46, A01G 7/06, A01P 21/00, C07C 331/30

(54) **PLANT STOMATAL OPENING REGULATOR**

(30) Priority: 26.04.2022 JP 2022072557
(71) Applicant: NATIONAL UNIVERSITY CORPORATION TOKAI NATIONAL HIGHER EDUCATION AND RESEARCH SYSTEM, Nagoya-shi, Aichi 464-8601 (JP); Kwansei Gakuin Educational Foundation, Nishinomiya-shi, Hyogo 662-8501 (JP)
(72) Inventor: KINOSHITA, Toshinori, Nagoya-shi, Aichi 464-8601 (JP); AIHARA, Yusuke, Nagoya-shi, Aichi 464-8601 (JP); SATO, Ayato, Nagoya-shi, Aichi 464-8601 (JP); TOH, Shigeo, Nagoya-shi, Aichi 464-8601 (JP); MURAKAMI, Kei, Sanda-shi, Hyogo 669-1330 (JP); YE, Wenxiu, Nagoya-shi, Aichi 464-8601 (JP); TODA, Yosuke, Nagoya-shi, Aichi 464-8601 (JP); ITAMI, Kenichiro, Nagoya-shi, Aichi 464-8601 (JP); GOTO, Kanna, Sanda-shi, Hyogo 669-1330 (JP); MAEDA, Bumpei, Sanda-shi, Hyogo 669-1330 (JP)
(74) Representative: Hoffmann Eitle
(86) International application number: PCT/JP2023/016086
(87) International publication number: WO 2023/210570

(57) **Abstract**

Provided is a plant stomatal opening regulator. The plant stomatal opening regulator comprises at least one member selected from the group consisting of a compound represented by formula (1), a salt thereof, and a solvate thereof.

## Description

### Technical Field

The present invention relates to a plant stomatal opening regulator and the like.

### Background Art

Terrestrial higher plants regulate the amount of carbon dioxide uptake and transpiration required for photosynthesis through the opening of stomata in the leaf epidermis. For example, it is known that when exposed to water shortage, plants close their stomata and suppress transpiration in order to maintain moisture within the plant body. It is also known that stomata open in response to light, promoting the uptake of carbon dioxide necessary for photosynthesis. Therefore, the artificial regulation of stomatal opening is expected to have effects such as promoting photosynthesis, facilitating growth, and improving drought tolerance.

As an example of the artificial regulation of stomatal opening, a method of overexpressing plasma membrane H⁺-ATPase (AHA2) in guard cells has been reported (PTL 1 and NPL 1). This method can promote stomatal opening and thus can also promote the photosynthetic rate and plant growth. There has also been reported a method for improving drought tolerance by overexpressing the magnesium-chelatase H subunit in guard cells and promoting stomatal closure (NPL 2).

Since these methods are based on genetic recombination technology, the following is necessary:
1. It is always necessary to establish and optimize recombination technology for species for which genetic recombination technology has not been established.
2. It takes time to create genetically modified organisms (GMOs). For example, it takes about six months to produce the current generation of transgenic poplar, and more than six months to obtain the next generation of GMO rice seeds.
3. Approval is required for each GMO created.

On the other hand, substances that can regulate stomatal opening by application to already growing plants would be extremely useful because they would not require the creation and approval of GMOs for each plant.

PTL 2 has reported that a compound called SCL1 has stomatal opening regulatory action.

### Citation List

### Patent Literature

PTL 1: WO2014/142334
PTL 2: JP2020-055767A

### Non-patent Literature

NPL 1: PNAS, January 7, 2014, vol. 111, no. 1, pp. 533-538
NPL 2: Front Plant Sci, October 30, 2013, vol. 4, Article 440

### Summary of Invention

### Technical Problem

An object of the present invention is to provide a plant stomatal opening regulator.

### Solution to Problem

As a result of intensive research in light of the above object, the present inventors found that compounds represented by formula (1) each have stomatal opening regulatory action. Upon further research based on this finding, the present invention has been completed. That is, the present invention includes the following aspects.

### Item 1.

A plant stomatal opening regulator comprising at least one member selected from the group consisting of a compound represented by formula (1), a salt thereof, and a solvate thereof: 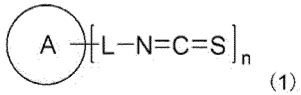 wherein A represents an optionally substituted ring, L are the same or different and each represents a linker, and n represents an integer of 1 to 6.

### Item 2.

The plant stomatal opening regulator according to Item 1, wherein A is a 5- or 6-membered monocyclic ring.

### Item 3.

The plant stomatal opening regulator according to Item 1 or 2, wherein A is an unsubstituted ring or a ring substituted with at least one member selected from the group consisting of an optionally substituted alkyl group, an optionally substituted alkoxy group, an optionally substituted alkoxycarbonyl group, an optionally substituted aryl group, a cyano group, a halogen atom, a nitro group, an optionally substituted amino group, an optionally substituted amide group, an optionally substituted ketone group, an optionally substituted ammonio group, and an optionally substituted thio group.

### Item 4.

The plant stomatal opening regulator according to any one of Items 1 to 3, wherein A has 0 to 3 substituents.

### Item 5.

The plant stomatal opening regulator according to any one of Items 1 to 4, wherein L is an alkylene group optionally substituted with an alkyl group and/or an aryl group.

### Item 6.

The plant stomatal opening regulator according to any one of Items 1 to 5, wherein:
A is a 5- or 6-membered monocyclic ring,
L is an alkylene group optionally substituted with an alkyl group and/or an aryl group, and
when A is a benzene ring and n is 1, A is a benzene ring substituted with at least one member selected from the group consisting of an optionally substituted aryl group, a cyano group, an iodine atom, and an optionally substituted alkoxycarbonyl group, and/or L is an alkylene group substituted with an alkyl group and/or an aryl group.

### Item 7.

The plant stomatal opening regulator according to any one of Items 1 to 6, which is a plant stomatal opening inhibitor.

### Item 8.

A drought tolerance improving agent comprising the plant stomatal opening regulator according to any one of Items 1 to 7.

### Item 9.

The drought tolerance improving agent according to Item 8, for use in the suppression of wilting.

### Item 10.

A drought tolerance improving method comprising applying the plant stomatal opening regulator according to any one of Items 1 to 7 to a plant.

### Item 11.

The drought tolerance improving method according to Item 10, comprising bringing the plant stomatal opening regulator according to any one of Items 1 to 7 into contact with plant stomata.

### Item 12.

A plasma membrane proton pump phosphorylation inhibitor comprising at least one member selected from the group consisting of a compound represented by formula (1), a salt thereof, and a solvate thereof: 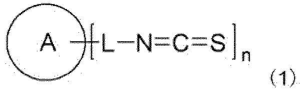 wherein A represents an optionally substituted ring, L represents a linker, and n represents an integer of 1 to 6.

### Item 13.

A plant covering or support material comprising at least one member selected from the group consisting of a compound represented by formula (1), a salt thereof, and a solvate thereof: 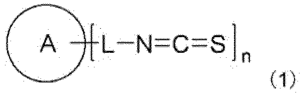 wherein A represents an optionally substituted ring, L represents a linker, and n represents an integer of 1 to 6.

### Item 14.

A compound represented by formula (1X), a salt thereof, or a solvate thereof: 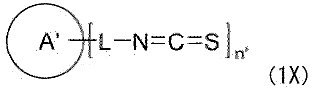 wherein A' represents an optionally substituted non-aromatic ring, L are the same or different and each represents a linker, and n' represents an integer of 2 to 6.

The items specifying the above regulator can be replaced with, for example, use of the active ingredient of the present invention for the production of the regulator, use of the active ingredient of the present invention for the application of the regulator, or the active ingredient of the present invention for use in the application of the regulator.

### Advantageous Effects of Invention

The present invention can provide a plant stomatal opening regulator and a compound serving as an active ingredient of the regulator. Also provided are a drought tolerance improving agent, a plasma membrane proton pump phosphorylation inhibitor, and the like.

### Brief Description of Drawings

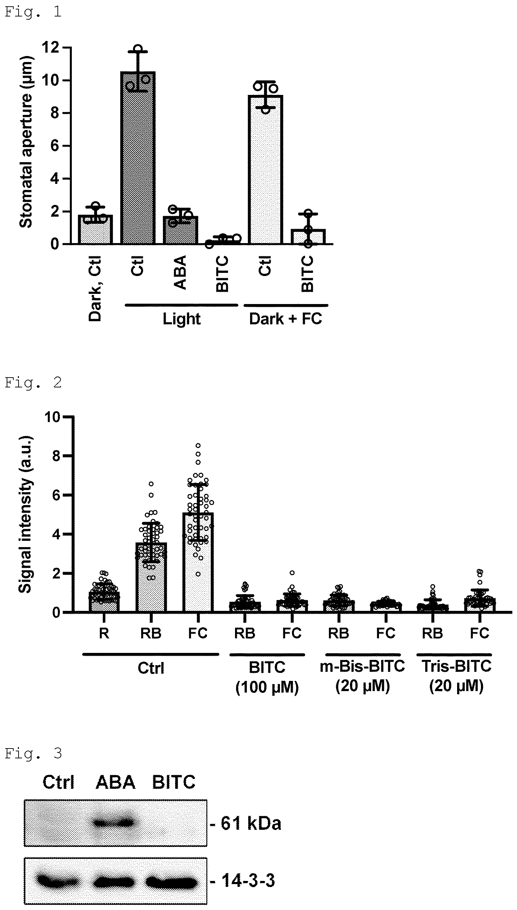

Fig. 1 shows the measurement results of Test Example 2. The vertical axis indicates the stomatal aperture. On the horizontal axis, "Dark" indicates a dark-treated group, "Light" indicates a light-treated group, "Ctl" indicates a case where a test solution containing no test compound was used, "ABA" indicates a case where a test solution containing abscisic acid as the test compound was used, "BITC" indicates a case where a test solution containing BITC (compound 1) as the test compound was used, and "+FC" indicates a case where a test solution containing fusicoccin in addition to the test compound was used.
Fig. 2 shows the measurement results of Test Example 3. The vertical axis indicates the signal intensity of phosphorylated plasma membrane proton pumps. On the horizontal axis, "Ctrl" indicates a case where no test compound was applied, "BITC," "m-Bis-BITC," and "Tris-BITC" indicate cases where respective test compounds were applied, "R" indicates a case of treatment with red light only, "RB" indicates a case of treatment with red light and blue light, and "FC" indicates a case where fusicoccin was applied.
Fig. 3 shows the measurement results of Test Example 4. In the upper part of the photographs, "Ctrl" indicates a case where no test compound was applied, "BITC" indicates a case where BITC (compound 1) was applied, and "ABA" indicates a case where abscisic acid was applied. The right side of the photographs shows the detected proteins, with "61 kDa" indicating the ABA-responsive kinase substrate, and "14-3-3" indicating the 14-3-3 protein, which is a loading control.

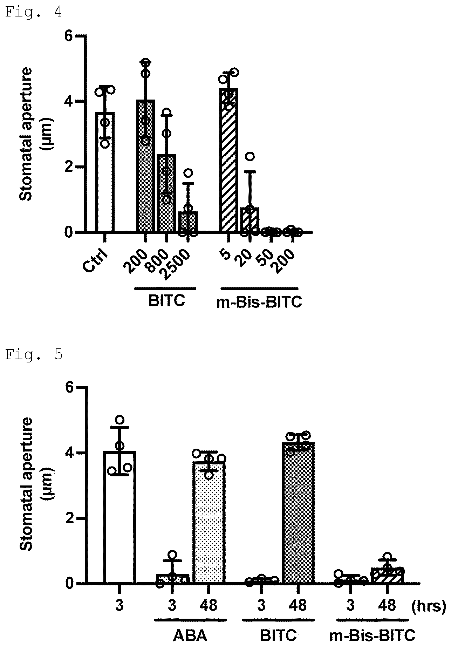

Fig. 4 shows the results of measuring the stomatal aperture after 3 hours of incubation in Test Example 5. The vertical axis indicates the stomatal aperture. On the horizontal axis, "Ctrl" indicates a case where a test solution containing no test compound was used, "BITC" and "m-Bis-BITC" indicate cases where test solutions containing respective test compounds were used, and the numerical values indicate the concentrations (unit: µM) of the test compounds in the test solutions.
Fig. 5 shows the results of measuring the stomatal aperture after 3 hours or 48 hours of incubation in Test Example 5. The vertical axis indicates the stomatal aperture. On the horizontal axis, the numbers indicate the incubation time, and "ABA," "BITC," and "m-Bis-BITC" indicate cases where test solutions containing respective test compounds were used. The leftmost column shows a case where a test solution containing no test compound was used. The test compound concentrations of the test solutions were 100 µM for ABA, 2500 µM for BITC, and 50 µM for m-Bis-BITC.

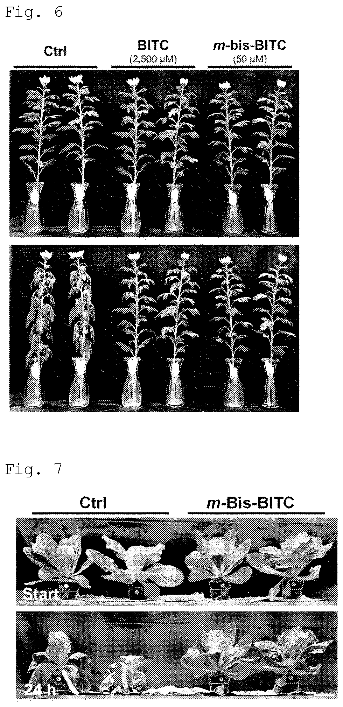

Fig. 6 shows photographs of the appearance of Test Example 6. "Ctrl" indicates a case where a test solution containing no test compound was used, "BITC" and "m-Bis-BITC" indicate cases where test solutions containing respective test compounds were used, and the numerical values indicate the concentrations of the test compounds in the test solutions. The upper photograph shows the appearance at the start of incubation in a dehydrated state, and the lower photograph shows the appearance at the end of incubation in a dehydrated state.
Fig. 7 shows photographs of the appearance of Test Example 7. "Ctrl" indicates a case where a test solution containing no test compound was used, and "m-Bis-BITC" indicates a case where a test solution containing a test compound was used. The upper photograph shows the appearance at the start of culture in a dehydrated state, and the lower photograph shows the appearance at the end of culture in a dehydrated state.

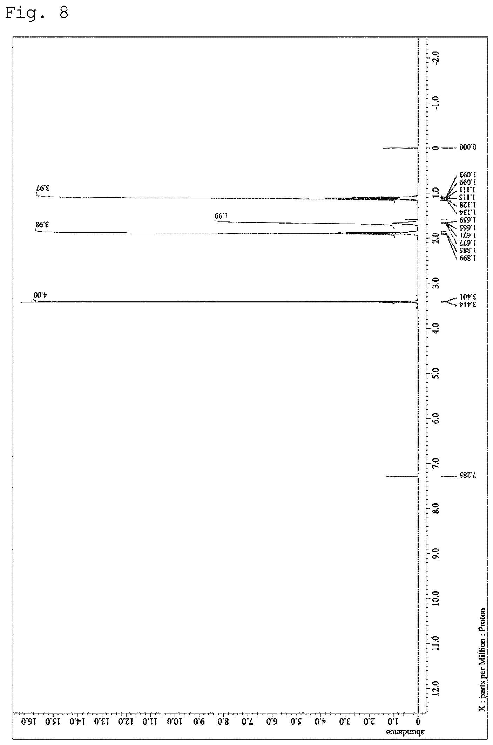

Fig. 8 shows the ¹H NMR (500 MHz, CDCl₃) results of compound 27 synthesized in Test Example 1-1-3.

### Description of Embodiments

In the present specification, the terms "comprise" and "contain" include the concepts of "comprise," "contain," "essentially consist of," and "consist of."

An aspect of the present invention relates to a plant stomatal opening regulator (also referred to as "the plant stomatal opening regulator of the present invention" in the present specification) comprising at least one member selected from the group consisting of a compound represented by formula (1) (also referred to as "the compound of the present invention" in the present specification), a salt thereof, and a solvate thereof (also referred to as "the active ingredient of the present invention" in the present specification): 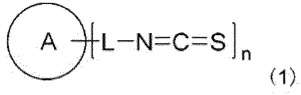 The compound of the present invention, the active ingredient of the present invention, and use thereof will be described below.

### 1. Active Ingredient

A represents an optionally substituted ring. The term "substituted" as mentioned herein does not include substitution with -L-N=C=S.

The ring is not particularly limited, and includes any of an aromatic ring composed only of hydrocarbons, an aromatic ring containing a heteroatom other than carbon and hydrogen (heterocyclic ring), a non-aromatic ring composed only of hydrocarbons (alicyclic hydrocarbon), and a non-aromatic ring containing a heteroatom other than carbon and hydrogen (heterocyclic ring). The heteroatom contained in the heterocyclic ring is not particularly limited, and examples include sulfur, nitrogen, and oxygen atoms. The ring may be a monocyclic ring (e.g., a 4- to 8-membered ring) or a fused ring (e.g., a bicyclic or tricyclic ring). Examples of aromatic rings include monocyclic rings, such as benzene, thiophene, furan, pyrrole, imidazole, pyrazole, oxazole, isoxazole, thiazole, triazole, tetrazole, pyridine, pyrazine, pyrimidine, pyridazine, and triazine; bicyclic rings, such as naphthalene, benzothiophene, benzofuran, indole, benzimidazole, indazole, benzoxazole, benzothiazole, isobenzofuran, isoindole, purine, quinoline, isoquinoline, quinoxaline, quinazoline, cinnoline, and phthalazine; tricyclic rings, such as anthracene and phenanthrene; and the like. Examples of non-aromatic rings include cycloalkanes, such as cyclopropane, cyclobutane, cyclopentane, cyclohexane, cycloheptane, cyclooctane, cyclononane, cyclodecane, cycloundecane, and cyclododecane; cycloalkenes, such as cyclopropene, cyclobutene, cyclopropene, cyclohexene, cycloheptene, and cyclooctene; bicyclic alkanes, such as bicycloundecane and decahydronaphthalene; bicyclic alkenes, such as bicycloundecane and decahydronaphthalene; and the like.

The ring is preferably a monocyclic ring, in terms of plant stomatal opening regulatory action. The ring is preferably 5- or 6-membered, in terms of plant stomatal opening regulation. When the ring is a heterocyclic ring, the heteroatoms contained in the heterocyclic ring preferably include a sulfur atom in terms of plant stomatal opening regulation, and more preferably a sulfur atom alone. The ring is preferably an aromatic ring, in terms of plant stomatal opening regulatory action.

The ring is an unsubstituted ring or a substituted ring. The substituents of the ring are not particularly limited, and examples include an optionally substituted alkyl group, an optionally substituted alkoxy group, an optionally substituted alkoxycarbonyl group, an optionally substituted aryl group, a cyano group, a halogen atom, a nitro group, an optionally substituted amino group, an optionally substituted amide group, an optionally substituted ketone group, an optionally substituted ammonio group, an optionally substituted thio group, and the like. More preferred among these are an optionally substituted alkyl group, an optionally substituted alkoxy group, an optionally substituted alkoxycarbonyl group, an optionally substituted aryl group, a cyano group, and a halogen atom; and particularly preferred are an optionally substituted aryl group, a cyano group, an iodine atom, and an optionally substituted alkoxycarbonyl group.

The alkyl group may be liner, branched, or cyclic. In terms of plant stomatal opening regulatory action, the alkyl group is preferably linear or branched, and more preferably linear. The number of carbon atoms in the alkyl group is not particularly limited, and is, for example, 1 to 8, in terms of plant stomatal opening regulatory action, preferably 1 to 6, more preferably 1 to 4, even more preferably 1 or 2, and particularly preferably 1. Specific examples of the alkyl group include methyl, ethyl, n-propyl, isopropyl, n-butyl, isobutyl, tert-butyl, sec-butyl, n-pentyl, neopentyl, n-hexyl, and 3-methylpentyl groups. The alkyl group may be substituted with a halogen atom, such as fluorine, chlorine, bromine, or iodine.

The alkoxy group may be liner or branched. In terms of plant stomatal opening regulatory action, the alkoxy group is preferably liner. The number of carbon atoms in the alkoxy group is not particularly limited, and is, for example, 1 to 8, in terms of plant stomatal opening regulatory action, preferably 1 to 6, more preferably 1 to 4, even more preferably 1 or 2, and particularly preferably 1. Specific examples of the alkoxy group include methoxy, ethoxy, n-propoxy, isopropoxy, n-butoxy, isobutoxy, sec-butoxy, and tert-butoxy groups. The alkoxy group may be substituted with a halogen atom, such as fluorine, chlorine, bromine, or iodine.

The alkoxycarbonyl group is a group formed by linking an alkoxy group and a carbonyl group. The alkoxy group is as described above.

The aryl group is not particularly limited; however, in terms of plant stomatal opening regulatory action, preferred is one having 6 to 12 carbon atoms, and more preferred is one having 6 to 8 carbon atoms. The aryl group may be either monocyclic or polycyclic (e.g., bicyclic or tricyclic), but is preferably monocyclic in terms of plant stomatal opening regulatory action. Specific examples of the aryl group include phenyl, naphthyl, biphenyl, pentalenyl, indenyl, anthranyl, tetracenyl, pentacenyl, pyrenyl, perylenyl, fluorenyl, and phenanthryl groups; and preferably a phenyl group. The aryl group may be substituted with a halogen atom, such as fluorine, chlorine, bromine, or iodine.

The halogen atom is not particularly limited, and examples include a fluorine atom, a chlorine atom, a bromine atom, an iodine atom, and the like.

The hydrogen atoms in the amino group (-NH₂), amide group (-CO-NH₂), ketone group (-CO-H), ammonio group (-NH₃), and thio group (-SH) may be replaced by a hydrocarbon group, such as an alkyl group or an aryl group. The alkyl and aryl groups are as explained above.

In terms of plant stomatal opening regulatory action, the substituent on the ring is particularly preferably an optionally substituted aryl group.

The number of substituents (substituents other than -L-N=C=S) on the ring is not particularly limited, but is, for example, 0 to 6. In terms of plant stomatal opening regulatory action, the number of substituents is preferably 0 to 3, more preferably 0 to 2, and particularly preferably 0 or 1.

In the compound of the present invention, the optionally substituted ring represented by A is such that a hydrogen atom on the ring is replaced by n (1 to 6) -L-N=C=S. As a result, the compound of the present invention can exhibit excellent plant stomatal opening regulatory action.

L are the same or different and each represents a linker.

The linker contains a chain structure. The chain structure is not particularly limited, but examples of the chain structure include a chain structure having, for example, 1 to 20 atoms constituting the main chain, and in terms of plant stomatal opening regulatory action, preferably 1 to 15, more preferably 1 to 12, even more preferably 1 to 10, still more preferably 1 to 8, especially preferably 1 to 6, and particularly preferably 1 or 2. Examples of the main chain constituent atoms include carbon, oxygen, sulfur, and nitrogen atoms. A part or the whole of the chain structure may be an alkylene group or a heteroalkylene group. The chain structure may be either liner or branched, but is preferably liner in terms of plant stomatal opening regulatory action. Further, the chain structure can include a partial structure, such as -O-, -C(=O)-O-, -CO-NH-, -C(=O)-, -NH-, or - S(=O)₂-, on the main chain. The chain structure may be substituted with an alkyl group and/or an aryl group. The alkyl and alkylene groups are the same as those described above.

In terms of plant stomatal opening regulatory action, the linker is preferably an alkylene group optionally substituted with an alkyl group and/or an aryl group, and particularly preferably an alkylene group substituted with an alkyl group and/or an aryl group.

n represents an integer of 1 to 6.

In terms of plant stomatal opening regulatory action, n is particularly preferably 2 or more. The upper limit of n is, for example, 5, 4, or 3. In one aspect of the present invention, in terms of plant stomatal opening regulatory action, n is preferably 2 to 5, more preferably 2 to 4, and even more preferably 2 or 3. In addition, in terms of plant stomatal opening regulatory action when applied to intact leaves, n is particularly preferably 2.

In one aspect of the present invention, preferably, A is a 5- or 6-membered monocyclic ring, L is an alkylene group optionally substituted with an alkyl group and/or an aryl group, and when A is a benzene ring and n is 1, A is a benzene ring substituted with at least one member selected from the group consisting of an optionally substituted aryl group, a cyano group, an iodine atom, and an optionally substituted alkoxycarbonyl group, and/or L is an alkylene group substituted with an alkyl group and/or an aryl group.

In one aspect of the present invention, formula (1) is preferably formula (1X):

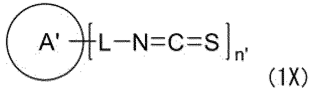

A' represents an optionally substituted non-aromatic ring, L are the same or different and each represents a linker, and n' represents an integer of 2 to 6.

In one aspect of the present invention, formula (1) is preferably formula (1Y):

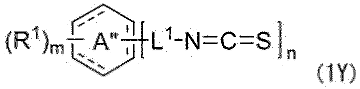

In one aspect of the present invention, formula (1) is preferably formula (1A):

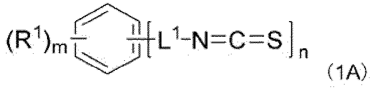

A" represents a benzene ring or cyclohexane. A" is a benzene ring in one aspect, and is cyclohexane in another aspect.

L¹s are the same or different and each represents an alkylene group optionally substituted an alkyl group and/or an aryl group.

R¹s are the same or different and each represents an optionally substituted alkyl group, an optionally substituted alkoxy group, an optionally substituted alkoxycarbonyl group, an optionally substituted aryl group, a cyano group, a halogen atom, a nitro group, an optionally substituted amino group, an optionally substituted amide group, an optionally substituted ketone group, an optionally substituted ammonio group, or an optionally substituted thio group. Especially preferred among these (in an aspect, when A" is a benzene ring, or when A" is a benzene ring and n is 1) are an optionally substituted aryl group, a cyano group, an iodine atom, and an optionally substituted alkoxycarbonyl group; and particularly preferred is an optionally substituted aryl group.

The groups represented by L¹ and R¹ are as explained above.

n is the same as above.

m is a number satisfying 0≤m≤6-n. In terms of plant stomatal opening regulatory action, m is preferably 0 to 3, more preferably 0 to 2, and particularly preferably 0 or 1.

In one aspect of the present invention, formula (1) is preferably formula (1Z):

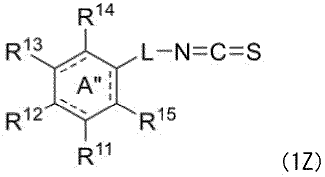

In one aspect of the present invention, formula (1) is preferably formula (1B):

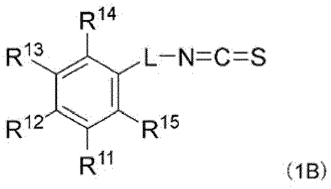

A" is a benzene ring or cyclohexane. A" is a benzene ring in one aspect, and is cyclohexane in another aspect.

R¹¹, R¹², R¹³, R¹⁴, and R¹⁵ are the same or different and each represents a hydrogen atom, -L-N=C=S, an optionally substituted alkyl group, an optionally substituted alkoxy group, an optionally substituted alkoxycarbonyl group, an optionally substituted aryl group, a cyano group, a halogen atom, a nitro group, an optionally substituted amino group, an optionally substituted amide group, an optionally substituted ketone group, an optionally substituted ammonio group, or an optionally substituted thio group. Especially preferred among these are an optionally substituted aryl group, a cyano group, an iodine atom, and an optionally substituted alkoxycarbonyl group; and particularly preferred is an optionally substituted aryl group.

L are the same or different and each represents a linker.

The groups represented by L¹, R¹¹, R¹², R¹³, R¹⁴, and R¹⁵ are as explained above.

In an aspect (aspect 1) of the present invention, at least one of R¹² and R¹³ is -L-N=C=S, and when R¹³ is -L-N=C=S, at least one of R¹¹, R¹², R¹⁴, and R¹⁵ is a group other than a hydrogen atom. In aspect 1, R¹⁴ and R¹⁵ are preferably hydrogen atoms. In aspect 1, preferably one of R¹² and R¹³ is -L-N=C=S, and the other is a hydrogen atom. In aspect 1, when R¹² is -L-N=C=S, R¹¹ is preferably a hydrogen atom. In aspect 1, when R¹³ is -L-N=C=S, R¹¹ is preferably an alkyl group or -L-N=C=S.

The compound represented by formula (1) may include stereoisomers and optical isomers; however, these are not particularly limited.

The salt of the compound represented by formula (1) is not particularly limited as long as it is an agriculturally acceptable salt. As the salt, either an acidic salt or a basic salt can be used. Examples of acidic salts include inorganic acid salts, such as hydrochloride, hydrobromide, sulfate, nitrate, and phosphate; and organic acid salts, such as acetate, propionate, tartrate, fumarate, maleate, malate, citrate, methanesulfonate, and paratoluenesulfonate. Examples of basic salts include alkali metal salts, such as sodium salts and potassium salts; alkaline earth metal salts, such as calcium salts and magnesium salts; salts with ammonia; and salts with organic amines, such as morpholine, piperidine, pyrrolidine, monoalkylamine, dialkylamine, trialkylamine, mono(hydroxyalkyl)amine, di(hydroxyalkyl)amine, and tri(hydroxyalkyl)amine.

The compound represented by formula (1) can be a hydrate or a solvate. Examples of solvents include agriculturally acceptable organic solvents (e.g., ethanol, glycerol, and acetic acid) and the like.

As the compound represented by formula (1), for example, a known compound can be obtained (e.g., by purchasing a commercially available product) and used. Examples of known compounds include the compounds shown in Tables 1 to 3. In the tables, -NCS represents an isothiocyanate group (-N=C=S).

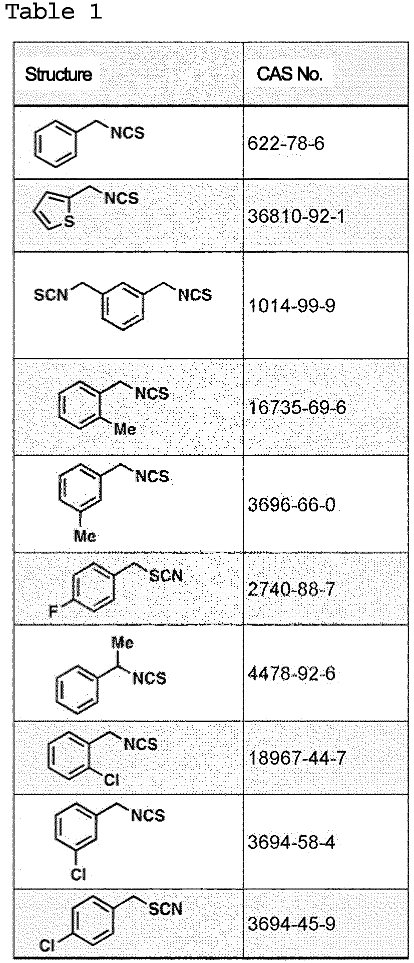

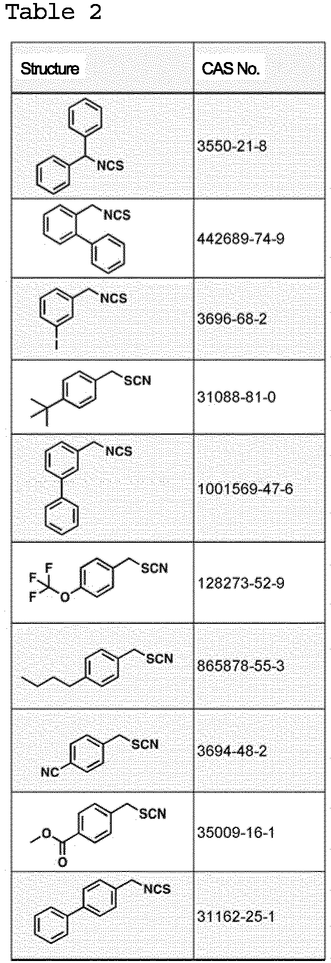

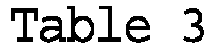

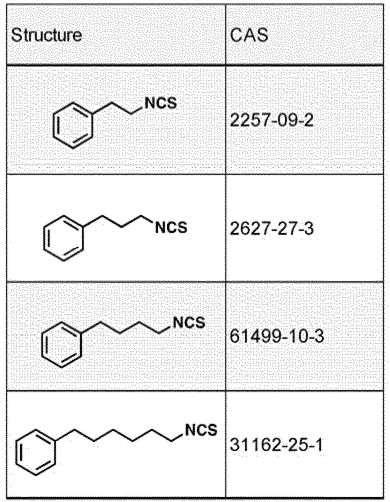

The compound represented by formula (1) can be produced by a method comprising reacting a compound represented by formula (a) (compound a): 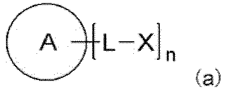
wherein A, L, and n are as defined above, and X represents a halogen atom;
with thiocyanate. Alternatively, when X is replaced by an amino group, the compound represented by formula (1) can be produced by reaction with a compound containing thione sulfur, such as di(1H-imidazol-1-yl)methanethione.

Compound a may be commercially available or may be synthesized according to a known method.

Examples of thiocyanate include potassium thiocyanate and sodium thiocyanate, and preferably potassium thiocyanate can be used.

In terms of yield, ease of synthesis, etc., the amount of thiocyanate used is generally preferably 3 to 30 mol, and more preferably 7 to 15 mol, per mol of compound a.

This reaction is generally carried out in the presence of a reaction solvent. The reaction solvent is not particularly limited, and examples include dimethylformamide, dichloromethane, acetonitrile, tetrahydrofuran, acetone, toluene, and the like; and preferably dimethylformamide and the like. The solvents may be used singly or in combination of two or more.

The reaction can be carried out under heating, at room temperature, or under cooling, generally at a temperature of 0 to 120°C. The reaction temperature is particularly preferably 70 to 100°C. The reaction time is not particularly limited, but can be generally 30 minutes to 60 hours.

The progress of the reaction can be followed by a conventional method, such as chromatography. After the reaction is completed, the solvent is distilled off, and the product can be isolated and purified by a general method, such as chromatography or recrystallization. The structure of the product can be identified by elemental analysis, MS (ESI-MS) analysis, IR analysis, ¹H-NMR, ¹³C-NMR, or the like.

### 2. Use

The active ingredient of the present invention has plant stomatal opening regulatory action. The regulation of stomatal opening can be used to regulate photosynthesis and even plant growth. Therefore, at least one member selected from the group consisting of the compound represented by formula (1), a salt thereof, and a solvate thereof can be used as an active ingredient for photosynthesis regulators, plant growth regulators, and the like.

In particular, the compound represented by formula (1) has the effect of inhibiting the stomatal opening of plants (particularly in response to light or drugs (e.g., fusicoccin)). It is also known that the inhibition of stomatal opening in plants reduces transpiration and maintains the water content within the plant. Therefore, at least one member selected from the group consisting of the compound represented by formula (1), a salt thereof, and a solvate thereof can be used as an active ingredient for plant stomatal opening inhibitors, drought tolerance improving agents, wilting suppressors, freshness preserving agents, and the like. It is also known that pathogenic microorganisms can enter plants through stomatal openings. Therefore, at least one member selected from the group consisting of the compound represented by formula (1), a salt thereof, and a solvate thereof can be used as an active ingredient for disease resistance improving agents and the like.

It is considered that the inhibition of phosphorylation of the plasma membrane proton pump is part of the mechanism of the plant stomatal opening regulatory action of the compound represented by formula (1). Therefore, at least one member selected from the group consisting of the compound represented by formula (1), a salt thereof, and a solvate thereof can be used as an active ingredient for plasma membrane proton pump phosphorylation inhibitors. The target plasma membrane proton pump is not particularly limited as long as it is expressed in guard cells. For example, in the case of *Arabidopsis thaliana,* examples include AHA1 (AT2G18960), AHA2 (AT4G30190), AHA3 (AT5G57350), AHA4 (AT3G47950), AHA5 (AT2G24520), AHA6 (AT2G07560), AHA7 (AT3G60330), AHA8 (AT3G42640), AHA9 (AT1G80660), AHA10 (AT1G17260), AHA11 (AT5G62670), and the like. In the case of rice, examples include OSA1 (LOC_Os03g48310), OSA2 (LOC_Os07g09340), OSA3 (LOC_Os12g44150), OSA4 (LOC_Os05g25550), OSA5 (LOC_Os08g14360), OSA6 (LOC_Os02g55400), OSA7 (LOC_Os04g56160), OSA8 (LOC_Os03g01120), OSA9 (LOC_Os03g08560), OSA10 (LOC_Os06g08310), and the like.

The target plant of the plant stomatal opening regulator of the present invention is not particularly limited as long as it is a plant having stomata. For example, the plant stomatal opening regulator of the present invention can be widely applied to plants such as angiosperms (dicotyledons, monocotyledons, etc.), gymnosperms, and ferns. Specific examples include *Solanaceae,* such as tomatoes, green peppers, chili peppers, and eggplants; cucurbits, such as cucumber, pumpkin, melon, and watermelon; vegetables, such as cabbage, broccoli, and Chinese cabbage; fresh or spicy vegetables, such as celery, parsley, and lettuce; alliums, such as leeks, onions, and garlic; legumes, such as soybeans, peanuts, green beans, peas, and adzuki beans; strawberries and other fruit vegetables; taproots, such as radish, turnip, carrot, and burdock; tubers, such as taro, cassava, potato, sweet potato, and Chinese yam; soft vegetables, such as asparagus, spinach, and mitsuba; flowers, such as lisianthus, stock, carnation, and chrysanthemum; grains, such as rice, wheat, barley, oats, and corn; grasses, such as bentgrass and Zoysia grass; oil crops, such as rapeseed and peanuts; sugar crops, such as sugarcane and sugar beet; fiber crops, such as cotton and rush; forage crops, such as clover, sorghum, and dent corn; deciduous fruit trees, such as apples, pears, grapes, and peaches; citrus fruits, such as unshu mandarins, lemons, and grapefruits; woody plants, such as rhododendron, azalea, and cedar; and the like.

The plant stomatal opening regulator of the present invention may consist of only the drug described above, but may also contain various additives in addition to the above drug depending on the dosage form, application mode, etc. described below. The content of the drug in the plant stomatal opening regulator can be appropriately determined depending on the dosage form, application mode, etc. described below, and can be, for example, in the range of 0.0001 to 100 mass%. As a more specific example, when the plant stomatal opening regulator of the present invention, which is a liquid, is brought into contact with stomata, the content of the drug is, for example, 1 to 5000 µM, preferably 5 to 3000 µM, more preferably 10 to 1000 µM, even more preferably 20 to 500 µM, and still even more preferably about 20 to 200 µM.

The dosage form of the plant stomatal opening regulator of the present invention is not particularly limited as long as it is an agriculturally acceptable dosage form. Examples include liquids, solids, powders, granules, granular formulations, wettable powders, flowables, emulsions, pastes, dispersions, and the like.

The additives are not particularly limited as long as they are agriculturally acceptable additives. Examples include carriers, surfactants, spreaders, spray adjuvants, thickeners, extenders, binders, vitamins, antioxidants, pH adjusters, evaporation inhibitors, pigments, and the like.

Particularly preferred among these are water repellency inhibitors for plants, such as silicone surfactants. The content of silicone surfactant may vary depending on the dosage form. For example, in the case of a liquid, the content is, for example, 0.01 to 0.1 mass%, and preferably 0.02 to 0.05 mass%, based on 100 mass% of the plant stomatal opening regulator of the present invention.

The application mode of the plant stomatal opening regulator of the present invention is not particularly limited as long as it is known as a mode of use of agricultural chemicals (or a mode to be developed in the future). Examples include spraying, dripping, application, or mixing or dissolving in the plant growth environment (in soil, water, solid medium, liquid medium, etc.). Since the target of action of the plant stomatal opening regulator of the present invention is stomata, it is preferable to apply the plant stomatal opening regulator of the present invention by bringing it into contact with plant stomata.

Further, the active ingredient of the present invention can be used by being contained in a plant covering or support material. From this point of view, the present invention in an aspect relates to a plant covering or support material comprising the active ingredient of the present invention.

The plant covering material is a material for covering a part or the whole of a plant, and is not particularly limited as long as it is intended. The shape of the plant covering material is not particularly limited, but is preferably in the form of a sheet. The raw material of the plant covering material is not particularly limited, and can be paper, plastic, or the like. Specific examples of the plant covering material include plant packaging sheets, agricultural sheets (sheets for agricultural greenhouses), and sheets for preserving the freshness of food (e.g., sheets used inside containers (e.g., lunch boxes) in which food, including plants, can be placed).

The plant support material is a material for supporting the plant so that the plant does not fall over, and is not particularly limited as long as it is intended. The plant support material is typically used by inserting or retaining the stem or root of the plant. The raw material of the plant support material is not particularly limited, and can be paper, plastic, or the like. Examples of the plant support material include porous bodies such as sponges and fiber aggregates, and more specifically solid culture media (e.g., rock wool pots) and water-absorbent sponges that are mainly used for floricultural purposes, such as flower arrangement.

The active ingredient of the present invention may constitute the surface layer of the covering or support material, or may be contained in the entire covering or support material.

The active ingredient of the present invention in the plant covering or support material can exhibit its effect on plants by bringing the covering or support material into contact with plants, or by being volatilized from the covering or support material and coming into contact with plants.

### Examples

The present invention is described in detail below based on Examples; however, the present invention is not limited to these Examples.

### Test Example 1. Stomatal Opening Regulatory Action Measurement

### Test 1

Compounds 1 to 27 (Tables 4 to 10) were prepared, and their stomatal opening regulatory action was measured. Compounds 3, 5, and 27 were synthesized as follows. At least compound 27 is a novel compound. The other compounds were synthesized in accordance with or based on previous reports, or were purchased commercially.

### Test Example 1-1. Synthesis of Compounds

### Test Example 1-1-1. Synthesis of Compound 3

Di(1H-imidazol-1-yl)methanethione (10 mmol, 5 equiv.) and DMF (15 mL) were added to a dry round-bottom flask equipped with a stir bar. A solution of Et₃N (4.8 mmol, 2.4 equiv.) and 1,4-phenylenedimethanamine (2.0 mmol, 1.0 equiv.) in DMF (5 mL) was added to the flask, and the mixture was stirred at 50°C overnight. The reaction mixture was quenched with H₂O (20 mL) and extracted with hexane/EtOAc = 1:1 (20 mL × 3). The organic layer was washed with 1M HCl aq. and water (3 times). Then, the organic layer was dried over Na₂SO₄, filtered, and concentrated. The crude product was purified by chromatography on silica gel to give compound 3 (221 mg, 1.00 mmol, 50%) as a pale yellow solid.
¹H NMR (500 MHz, CDCl₃) δ 4.73 (s, 4H), 7.35 (s, 4H); ¹³C NMR (126 MHz, CDCl₃) δ 48.37, 127.52, 132.83, 134.59; LRMS (GC-MS, EI): m/z = 220 calcd for C₁₀H₈N₂S₂220 [M⁺].

### Test Example 1-1-2. Synthesis of Compound 5

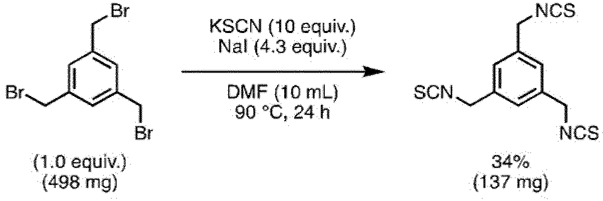

KSCN (1.36 g, 14 mmol, 10 equiv.), NaI (900 mg, 6 mmol, 4.3 equiv.), and 1,3,5-tris(bromomethyl)benzene (498 mg, 1.4 mmol, 1.0 equiv.) were added to a dry 100 ml round-bottom flask equipped with a stir bar. Next, dimethylformamide (10 mL) was added to the flask, and the mixture was stirred at 90°C for 24 hours. The reaction mixture was diluted with H₂O (20 mL) and extracted with diethyl ether (3 × 20 mL). The organic layer was dried over MgSO₄ and concentrated under reduced pressure. The crude product was purified by flash column chromatography (hexane/EtOAc = 100:0 to 70:30) on silica gel and preparative recycling gel permeation chromatography to give 1,3,5-tris(isothiocyanatomethyl)benzene (137 mg, 0.47 mmol, 34%).
¹H NMR (500 MHz, CDCl₃) δ 4.79 (s, 6H), 7.26 (s, 3H); ¹³C NMR (126 MHz, CDCl₃) δ 48.38, 125.29, 133.89, 136.39; LRMS (GC-MS): m/z= 291 calcd for 291 [M⁺].

### Test Example 1-1-3. Synthesis of Compound 27

Di(1H-imidazol-1-yl)methanethione (1.78 g, 10 mmol, 5 equiv.) and DMF (15 mL) were added to a dry 100 ml round-bottom flask equipped with a stir bar. Next, a solution of triethylamine (0.55 mL, 9.6 mmol, 4.8 equiv.) and trans-1,4-bis(aminomethyl)cyclohexane (280 mg, 2.0 mmol, 1.0 equiv.) in DMF (5 mL) was added to the flask, and the mixture was stirred at 50°C for 16 hours. The reaction mixture was diluted with H₂O (20 mL) and extracted with ethyl acetate (3 × 20 mL). The organic layer was dried over sodium sulfate, filtered, and concentrated under reduced pressure. The crude product was purified by flash column chromatography on silica gel to give trans-1,4-bis(isothiocyanatomethyl)cyclohexane (315 mg, 1.39 mmol, 70%) as a white solid.

Fig. 8 shows the results of ¹H NMR (500 MHz, CDCl₃). Chemical Formula: C₁₀H₁₄N₂S₂, Exact Mass: 226.06, Molecular Weight: 226.36.

### Test Example 1-2. Measurement Test

The test compounds (compounds 1 to 27) were dissolved at various concentrations to prepare stomatal aperture measurement solutions (5 mM MES-BTP [pH: 6.5], 50 mM KCl, 0.1 mM CaCl₂, 0.5% DMSO) as test solutions.

Part of the stem of *Commelina benghalensis* was inserted into a planter filled with mixed soil of vermiculite and peat moss, and grown indoors with natural light (room temperature: 25±3°C) for 2 to 4 weeks. The planter was placed in a dark room and subjected to dark treatment overnight. After the dark treatment, leaf disks with a diameter of 4 mm were cut from fully expanded leaves using a hole punch (Biospy Punch, Kai Medical).

Each test solution was dispensed into five wells of a 96-well plate at 50 µl/well, and the leaf disks were immersed therein, followed by incubation under light irradiation (50 µmol m⁻²s⁻¹ blue light) for 3 hours. After incubation, stomatal closure was evaluated under observation images (600x) using a stereofluorescence microscope (Leica M205FA). The minor diameter of about 20 stomata (hereinafter referred to as "stomatal aperture") was measured for each leaf disk. Based on the measured values of stomatal aperture at each concentration for each test compound, the 50% inhibitory concentration (IC₅₀) of stomatal aperture was calculated.

The results are shown in Tables 4 to 9. In the tables, -NCS represents an isothiocyanate group (-N=C=S), -Me represents a methyl group, and -Ph represents a phenyl group.

**Table 4**

| Structure | Compd. No. | IC₅₀ (*µ*M) |
|---|---|---|
| 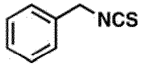 | 1 | 29.1 |
| 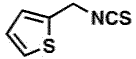 | 2 | 20.6 |
| 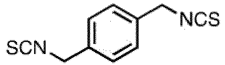 | 3 | 3.5 |
| 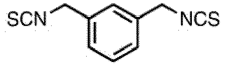 | 4 | 1.7 |
| 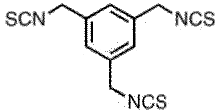 | 5 | 0.44 |

**Table 5**

| **Structure** | **Compd. No.** | p | IC₅₀ (*µ*M) |
|---|---|---|---|
| 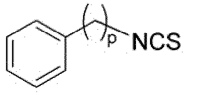 | 6 | 2 | 26.4 |
| | 7 | 3 | 36.6 |
| | 8 | 4 | 37.1 |
| | 9 | 6 | 35.7 |

**Table 6**

| **Structure** | **Compd. No.** | R^{a} | IC₅₀ (*µ*M) |
|---|---|---|---|
| 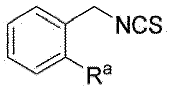 | 10 | - Me | 25.8 |
| | 11 | - Cl | 27.5 |
| | 12 | - Ph | 7.0 |

**Table 7**

| **Structure** | **Compd. No.** | R^{b} | IC₅₀ (*µ*M) |
|---|---|---|---|
| 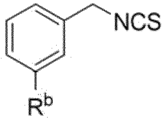 | 13 | - Me | 22.5 |
| | 14 | - Cl | 26.6 |
| | 15 | - I | 16.2 |
| | 16 | - Ph | 9.0 |

**Table 8**

| **Structure** | **Compd. No.** | R^{c} | IC₅₀ (*µ*M) |
|---|---|---|---|
| 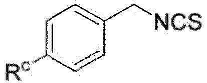 | 17 | - F | 30.2 |
| | 18 | - Cl | 23.7 |
| | 19 | - C[CH₃]₃ | 35.3 |
| | 20 | - OCF₃ | 36.6 |
| | 21 | - [CH₂]₃CH₃ | 48.6 |
| | 22 | - CN | 10.5 |
| | 23 | - COOCH₃ | 13.2 |
| | 24 | - Ph | 6.2 |

**Table 9**

| **Structure** | **Compd. No.** | R^{d} | IC₅₀ (*µ*M) |
|---|---|---|---|
| 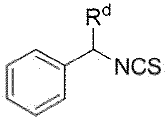 | 25 | - Me | 14.2 |
| | 26 | - Ph | 16.1 |

**Table 10**

| **Structure** | **Compd. No.** | IC₅₀ (*µ*M) |
|---|---|---|
| 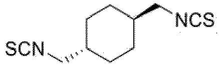 | 27 | 11.4 |

### Test Example 2. Stomatal Opening Regulatory Action Measurement

### Test 2

Compound 1 (BITC: benzyl isothiocyanate) (final concentration in the test solution: 100 µM) and abscisic acid (final concentration in the test solution: 20 µM) were used as test compounds. A test solution was also prepared by adding fusicoccin (final concentration: 10 µM) in addition to the test compound. The measurement test was performed in the same manner as in Test Example 1, except that in addition to the group incubated for 3 hours under light irradiation (150 µmol m⁻²s⁻¹ red light and 50 µmol m⁻²s⁻¹ blue light) (light irradiation group), a group incubated in a dark room for 3 hours in place of light irradiation (dark treatment group) was provided. The mean and standard deviation (SD) were calculated based on the measured values.

The results are shown in Fig. 1. It was found that compound 1 exhibited a more potent inhibitory effect on stomatal opening than abscisic acid, and also exhibited an inhibitory effect on stomatal opening induced by fusicoccin.

### Test Example 3. Analysis of Mechanism of Stomatal Opening

### Regulatory Action 1

Blue light- and fusicoccin-induced phosphorylation of the plasma membrane proton pump in guard cells from the epidermis of *Arabidopsis thaliana* was measured immunohistochemically as previously reported (Hayashi et al. (2011) Immunohistochemical detection of blue light-induced phosphorylation of the plasma membrane H+-ATPase in stomatal guard cells. Plant Cell Physiol. 52: 1238-1248). The test compounds used were compound 1 (BITC), compound 4 (m-Bis-BITC), and compound 5 (Tris-BITC). The concentration of compound 1 during action was 100 µM, the concentration of compound 4 during action was 20 µM, the concentration of compound 5 during action was 20 µM, and the concentration of fusicoccin during action was 10 µM.

The results are shown in Fig. 2. Compounds 1, 4, and 5 were found to have inhibitory effects on blue light-induced phosphorylation of the plasma membrane proton pump and on fusicoccin-induced phosphorylation of the plasma membrane proton pump.

### Test Example 4. Analysis of Mechanism of Stomatal Opening

### Regulatory Action 2

Abscisic acid (ABA)-induced phosphorylation of ABA-responsive kinase substrate (AKS) in guard cells from the epidermis of broad bean (*Vicia* faba) was measured by immunoblot analysis as previously reported (Takahashi et al. (2007) Protein Phosphorylation and Binding of a 14-3-3 Protein in Vicia Guard Cells in Response to ABA. Plant and Cell Physiology. 48: 1182-1191). The test compounds used were compound 1 (BITC) and abscisic acid. The concentration of compound 1 during action was 100 µM, and the concentration of abscisic acid during action was 20 µM.

The results are shown in Fig. 3. Compound 1 did not induce phosphorylation of the ABA-responsive kinase substrate.

Further analysis revealed that compound 1 had no effect on ABA-related responses in *Arabidopsis thaliana,* including inhibition of seed germination and induction of ABA-responsive genes (RAB18 and RD29B).

### Test Example 5. Stomatal Opening Regulatory Action Measurement

### Test 3

A bouquet of chrysanthemums (*Chrysanthemum*) was purchased and cultured in a water bottle at 24°C with a photoperiod of 16 h white light (100 µmol m⁻²s⁻¹)/8 h darkness for 2 days. After the dark period ended, the leaves were immersed in a test solution (0.033% Makupika (spreader, Isk Biosciences K.K.)) containing the test compound (compound 1 (BITC), compound 4 (m-Bis-BITC), or abscisic acid (ABA)) and incubated under white light (100 µmol m⁻²s⁻¹) for 3 hours or incubated with a photoperiod of 16 h light/16 h darkness (light → dark → light) for 48 hours. After incubation, the stomatal aperture was measured in the same manner as in Test Example 1.

The results are shown in Figs. 4 and 5. Compounds 1 and 4 were found to have an inhibitory effect on stomatal opening even in intact leaves. In addition, compound 4 was found to be excellent in the persistence of the stomatal opening inhibitory effect.

### Test Example 6. Drought Tolerance Test 1

After incubation for 3 hours in Test Example 5, the bouquet was dehydrated and incubated at 21°C for 90 minutes under 10 µmol m⁻²s⁻¹ white light at a relative humidity of 35 to 40%.

Fig. 6 shows photographs of the appearance at the start and end of the incubation in a dehydrated state. It was found that compounds 1 and 4 could improve drought tolerance and suppress wilting.

### Test Example 7. Drought Tolerance Test 2

A test solution (0.033% Makupika (spreader, Isk Biosciences K.K.)) containing compound 4 (m-Bis-BITC) was sprayed onto 4- to 5-week-old Chinese cabbage (*Brassica rapa*) growing in 110 ml of soil. The concentration of compound 4 in the test solution was 50 µM. Then, the water was depleted, and the plants were cultured for 24 hours in a greenhouse at 25°C at a relative humidity of 40 to 50% on a sunny day.

Fig. 7 shows photographs of the appearance at the start and end of the culture in a water-depleted state. It was found that compound 4 could improve drought tolerance and suppress wilting over a long period of time.

## Claims

1. A plant stomatal opening regulator comprising at least one member selected from the group consisting of a compound represented by formula (1), a salt thereof, and a solvate thereof: 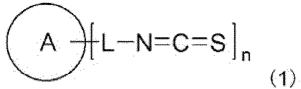 wherein A represents an optionally substituted ring, L are the same or different and each represents a linker, and n represents an integer of 1 to 6.

2. The plant stomatal opening regulator according to claim 1, wherein A is a 5- or 6-membered monocyclic ring.

3. The plant stomatal opening regulator according to claim 1 or 2, wherein A is an unsubstituted ring or a ring substituted with at least one member selected from the group consisting of an optionally substituted alkyl group, an optionally substituted alkoxy group, an optionally substituted alkoxycarbonyl group, an optionally substituted aryl group, a cyano group, a halogen atom, a nitro group, an optionally substituted amino group, an optionally substituted amide group, an optionally substituted ketone group, an optionally substituted ammonio group, and an optionally substituted thio group.

4. The plant stomatal opening regulator according to any one of claims 1 to 3, wherein A has 0 to 3 substituents.

5. The plant stomatal opening regulator according to any one of claims 1 to 4, wherein L is an alkylene group optionally substituted with an alkyl group and/or an aryl group.

6. The plant stomatal opening regulator according to any one of claims 1 to 5, wherein:
A is a 5- or 6-membered monocyclic ring,
L is an alkylene group optionally substituted with an alkyl group and/or an aryl group, and
when A is a benzene ring and n is 1, A is a benzene ring substituted with at least one member selected from the group consisting of an optionally substituted aryl group, a cyano group, an iodine atom, and an optionally substituted alkoxycarbonyl group, and/or L is an alkylene group substituted with an alkyl group and/or an aryl group.

7. The plant stomatal opening regulator according to any one of claims 1 to 6, which is a plant stomatal opening inhibitor.

8. A drought tolerance improving agent comprising the plant stomatal opening regulator according to any one of claims 1 to 7.

9. The drought tolerance improving agent according to claim 8, for use in the suppression of wilting.

10. A drought tolerance improving method comprising applying the plant stomatal opening regulator according to any one of claims 1 to 7 to a plant.

11. The drought tolerance improving method according to claim 10, comprising bringing the plant stomatal opening regulator according to any one of claims 1 to 7 into contact with plant stomata.

12. A plasma membrane proton pump phosphorylation inhibitor comprising at least one member selected from the group consisting of a compound represented by formula (1), a salt thereof, and a solvate thereof: 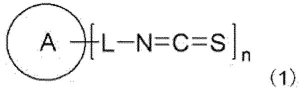 wherein A represents an optionally substituted ring, L represents a linker, and n represents an integer of 1 to 6.

13. A plant covering or support material comprising at least one member selected from the group consisting of a compound represented by formula (1), a salt thereof, and a solvate thereof: 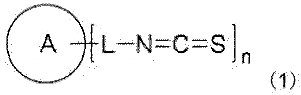 wherein A represents an optionally substituted ring, L represents a linker, and n represents an integer of 1 to 6.

14. A compound represented by formula (1X), a salt thereof, or a solvate thereof: 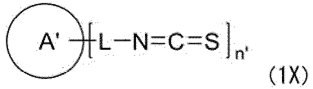 wherein A' represents an optionally substituted non-aromatic ring, L are the same or different and each represents a linker, and n' represents an integer of 2 to 6.
